(19) Europäisches Patentamt
European Patent Office
Office européen des brevets

(11) **EP 4 563 695 A1**

(12) **EUROPEAN PATENT APPLICATION**
published in accordance with Art. 153(4) EPC

(43) Date of publication:
**04.06.2025 Bulletin 2025/23**

(21) Application number: **23846622.1**

(22) Date of filing: **27.07.2023**

(51) International Patent Classification (IPC):
***C12N 1/04*** *(2006.01)* ***C12N 5/00*** *(2006.01)*

(52) Cooperative Patent Classification (CPC):
**C12N 1/04; C12N 5/00**

(86) International application number:
**PCT/JP2023/027582**

(87) International publication number:
**WO 2024/024892 (01.02.2024 Gazette 2024/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.07.2022 JP 2022120891**

(71) Applicant: **Sanyo-Onoda City Public University Corporation**
**Sanyo-Onoda-shi, Yamaguchi 756-0884 (JP)**

(72) Inventor: **HARA HORIGUCHI Michiko**
**Onoda-shi, Yamaguchi 756-0884 (JP)**

(74) Representative: **Hasegawa, Kan**
**Patentanwaltskanzlei Hasegawa**
**Untere Hauptstraße 56**
**85354 Freising (DE)**

# (54) COMPOSITION FOR CRYOPRESERVATION, CRYOPRESERVATION METHOD, AND FROZEN CELL OR BIOLOGICAL TISSUE

(57) It is an object of the present invention to provide a composition for cryopreservation while reducing cell damage due to cryopreservation and to provide a cryopreservation method while reducing cell damage due to cryopreservation. A composition for cryopreservation of a cell or biological tissue, the composition comprising a nanoparticle that comprises amphiphilic molecules as a constituent, wherein the amphiphilic molecules form a monolayer or bilayer, is produced.

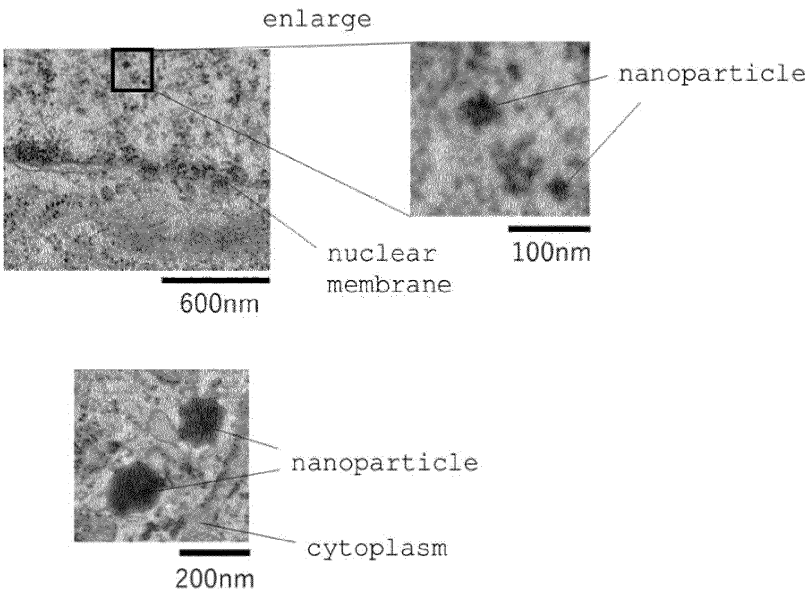


FIG. 2

Processed by Luminess, 75001 PARIS (FR)

EP 4 563 695 A1

## Description

### Technical Field

The present invention relates to a composition for cryopreservation, a cryopreservation method, and a frozen cell or biological tissue.

### Background Art

Regenerative medicine is a therapeutic method for treating diseases, injuries, and so on by using cells or the like. The regenerative medicine has been recently adopted as a therapeutic method for various diseases such as myocardial infarction, chronic arterial occlusion, and leukemia and injuries such as a spinal cord injury and a traumatic cartilage defect. Conventionally, cells and so on that are used for regenerative medicine are cryopreserved from the viewpoint of transportability, long-term storage, convenience, and so on. However, cryopreserved cells easily cause phenomena such as a reduction in the cell survival rate, cellular dysfunction (for example, in the case of stem cells, a reduction in the proportion of stem cells), and growth inhibition (hereinafter, these phenomena are generally also referred to as "cell damage"). The causes of this are believed to be that when cells are frozen, the water contained inside the cells crystalizes, and the resulting crystals damage the cell walls, organelles, and so on or that when cells are thawed, the water inside the cells is partially lost, and the shape of the cells changes.
As one cell cryopreservation method for freezing and preserving cells, as shown in described in Patent Document 1, a method for freezing cells in a buffer solution containing a low molecular weight compound such as dimethyl sulfoxide (DMSO) is known.

### Prior Art Documents

### Patent Documents

Patent Document 1: Japanese Patent No. 5773347

### Summary of the Invention

### Object to be Solved by the Invention

However, with the method using DMSO, the cell survival rate after cell thawing is low, and there is a room for improvement. The present invention has been made under such circumstances.
The object to be solved by one embodiment of the present invention is to provide a composition for cryopreserving cells or biological tissues while reducing cell damage due to cryopreservation.
The object to be solved by another embodiment of the present invention is to provide a method for cryopreserving a cell or biological tissues while reducing cell damage due to cryopreservation.

### Means to Solve the Object

Specific methods for solving the above objects include the following aspects:

<1> A composition for cryopreservation of cells or biological tissues, comprising a nanoparticle that comprises amphiphilic molecules as a constituent, wherein the amphiphilic molecules form a monolayer or bilayer;
<2> The composition for cryopreservation according to the above <1>, wherein the nanoparticle is a cationic nanoparticle;
<3> The composition for cryopreservation according to the above <1> or <2>, wherein the amphiphilic molecules are a Gemini-type surfactant;
<4> The composition for cryopreservation according to the above <1> or <2>, wherein the nanoparticle has a zeta-potential of 10 mV or more and 50 mV or less;
<5> The composition for cryopreservation according to the above <1> or <2>, wherein the nanoparticle has a volume average particle diameter of 30 nm or more and 300 nm or less;
<6> The composition for cryopreservation according to the above <1> or <2>, wherein the nanoparticle encapsulates ferulic acid or activated vitamin C;
<7> A method for cryopreserving a cell or biological tissue, comprising the steps of:

mixing cells or biological tissues with the composition for cryopreservation according to any one of the above <1> to <6> to introduce a nanoparticle into the cells or biological tissues; and
freezing the cells or biological tissues into which the nanoparticle has been introduced; and

<8> A frozen cell or biological tissue comprising a nanoparticle that comprises amphiphilic molecules as a constituent, wherein molecules of the amphiphilic molecules form a monolayer or bilayer.

**Effect of the Invention**

According to an embodiment of the present invention, a composition for cryopreservation of a cell or biological tissue while reducing damage of the cell or biological tissue due to cryopreservation is provided.
According to another embodiment of the present invention, a method for cryopreserving a cell or biological tissue while reducing damage of the cell or biological tissue due to cryopreservation is provided.

**Brief Description of Drawings**

[Figure 1] Figure 1 is a graph showing the distribution of particle diameters of a nanoparticle included in the composition of Example 3, in Test Example 1.
[Figure 2] Figure 2 is photographs showing the results of observation of nanoparticles introduced into cells with a transmission electron microscope, in Test Example 3.

**Mode of Carrying Out the Invention**

Modes for implementing the present invention will now be described. These descriptions and examples are illustrative of the present invention and are not intended to limit the scope of the present invention.
In this specification, the numerical range indicated using "to" indicates a range that includes the numerical values before and after "to" as the minimum and maximum values, respectively.

<Composition for cell cryopreservation>

A composition for cryopreservation of a cell or biological tissue according to the present embodiment (hereinafter, also referred to as "the present composition for cryopreservation") includes a nanoparticle that includes amphiphilic molecules as a constituent, wherein the amphiphilic molecules form a monolayer or bilayer (hereinafter, also referred to as "the present nanoparticle").
The present nanoparticle is introduced into a cell or cells constituting biological tissue by mixing the present composition for cryopreservation with the cells or biological tissues. That is, the present nanoparticle is introduced into the cells.
In a cell or biological tissue frozen with the present nanoparticle introduced therein, the present nanoparticle functions as a cushion (buffering function) against external force and temperature change applied to the cells, which suppresses crystallization and crystal growth of water included in the cells and makes it unlikely that the cell walls, organelles, and so on are damaged. In addition, the shape of a cell frozen with the present nanoparticle introduced therein, is easily maintained by the presence of the present nanoparticle when the cell is thawed. It is believed that as a result, damage due to cryopreservation is reduced.
Nanoparticles such as a liposome and a micelle have been conventionally used as technology for introducing a pharmacological component into an site of interest, such as a drug delivery system. In contrast, the invention according to the present disclosure focuses on the buffering function of the present nanoparticle itself exerted on a cell and is based on the findings that the physical properties possessed by the present nanoparticle effectively act to reduce cell damage that is caused by cryopreservation of the cell.

[Nanoparticle]

The present composition for cryopreservation includes a nanoparticle that includes amphiphilic molecules as a constituent, wherein molecules of the amphiphilic molecules form a monolayer or bilayer. The present composition for cryopreservation may include monolayer nanoparticles only, may include bilayer nanoparticles only, or may include both monolayer nanoparticles and bilayer nanoparticles.
The monolayer nanoparticle means a nanoparticle wherein amphiphilic molecules assembled so as to form a

monolayer. Specifically, examples of the monolayer nanoparticle include a micelle. The bilayer nanoparticle means a nanoparticle including a double membrane formed by a bilayer of amphiphilic molecules. Specifically, examples of the bilayer nanoparticle include (1) a liposome which is a vesicle including a lipid as the constituent and including a double membrane of the lipid and (2) a vesicle including a surfactant other than a lipid as the constituent and including a double membrane of the surfactant. The vesicle including a double membrane may be a small unilamellar liposome (SUV) or large unilamellar liposome (LUV) having one double membrane or may be multivesicular liposomes (MVLs) having two or more double membranes.

When the present nanoparticle includes a micelle, the micelle is preferably a micelle in which the hydrophilic group is on the outside and the hydrophobic group is on the inside.

The volume average particle diameter of the present nanoparticle can be 10 nm or more and 300 nm or less and may be 20 nm or more and 200 nm or less or 30 nm or more and 150 nm or less.

The smaller the volume average particle diameter of the present nanoparticle, the easier the particle to be distributed throughout cells, and the more likely the shape of the cells to be maintained when the cells are frozen.

When the present nanoparticle has a volume average particle diameter of 300 nm or less, the present nanoparticle can be more efficiently introduced into cells.

The method for controlling the volume average particle diameter of the present nanoparticle is not particularly limited, and examples thereof include methods such as sizing in which the particle diameter is adjusted with the aperture size of the filter that is used in manufacturing of the present nanoparticle. Specifically exemplified is extrusion treatment in which the present nanoparticle is allowed to pass through a filter with a pore by applying pressure, and the particle diameter is uniformized by physical shear force.

The present nanoparticle may be a cationic nanoparticle, a neutral nanoparticle, or an anionic nanoparticle and is more preferably a cationic nanoparticle. Usually, the potential inside a cell has a minus value (is negative) with respect to that outside the cell. Accordingly, when the present nanoparticle is a cationic nanoparticle, the present nanoparticle is easily introduced into a cell broadly and efficiently by interionic interaction. As a result, cell damage due to cryopreservation is further reduced.

The cationic nanoparticle is a nanoparticle having a zeta-potential of higher than 10 mV.

The neutral nanoparticle is a nanoparticle having a zeta-potential of -10 mV or more and 10 mV or less.

The anionic nanoparticle is a particle having a zeta-potential of less than -10 mV.

The present nanoparticle preferably has a zeta-potential of 10 mV or more and 80 mV or less, more preferably 15 mV or more and 70 mV or less, further preferably 20 mV or more and 60 mV or less, and particularly preferably 25 mV or more and 50 mV or less.

When the present nanoparticle has a zeta-potential of 10 mV or more, a membrane of the present nanoparticle is moderately positively charged. Accordingly, the present nanoparticle is efficiently introduced into a cell more easily, and cell damage due to cryopreservation is further reduced.

The zeta-potential and volume average particle diameter of the present nanoparticle can be measured, for example, as follows.

The present composition for cryopreservation is diluted by 100 times with phosphate buffered saline (PBS, pH 7.4), and 1 mL of the resulting solution is collected and filled in a 1-mL cell for measurement. The solution is subjected to measurement with a particle size and zeta-potential analyzer (ZETASIZER PRO, manufactured by Malvern) at room temperature (25°C).

The surface charge, i.e., the value of the zeta-potential, of the present nanoparticle is influenced by the charge of the amphiphilic molecules constituting the present nanoparticle. Accordingly, when a nanoparticle having a desired zeta-potential is prepared, amphiphilic molecules appropriately selected or modified depending on the desired zeta-potential is used. Such a method is exemplified as the method for preparing the present nanoparticle.

Examples of the amphiphilic molecules include a surfactant, and the surfactant may be a synthetic surfactant or a natural or organism-derived surfactant.

Examples of the synthetic surfactant include a Gemini-type surfactant. A Gemini-type surfactant includes a hydrophilic group and a hydrophobic group in one molecule and is a dimer surfactant or trimer surfactant having two or three surfactant units, respectively, linked to each other through the hydrophilic group by a spacer group.

Examples of the hydrophobic group constituting the Gemini-type surfactant include a straight-chain hydrocarbon chain having 8 to 24 carbon atoms, preferably 12 to 18 carbon atoms, and more preferably 12 or 16 carbon atoms, and is preferably a straight-chain saturated hydrocarbon chain from the viewpoint of the stability of the nanoparticle. The straight-chain hydrocarbon chains constituting two or three hydrophobic groups may be the same or different and preferably the same.

Examples of the hydrophilic group constituting the Gemini-type surfactant include a polar group and an ionic group. The elements that form an ion pair in the hydrophilic group can be appropriately coordinated depending on the aqueous solution to be used, and examples thereof include bromine (Br), chlorine (Cl), nitrogen (N), sodium (Na), phosphorus (P), boron (B), iodine (I), fluorine (F), sulfur (S), oxygen (O), carbon (C), beryllium (Be), iron (Fe), calcium (Ca),

and magnesium (Mg), and an arbitrary combination thereof can also be used.

The spacer is usually a hydrocarbon chain and preferably has a length for providing a sufficient distance such that hydrophobic groups can each independently act. The number of carbon atoms of the spacer is not particularly limited, and can be 2 to 12, preferably 2 to 8, further preferably 2 to 4, and most preferably 2 or 3.

Examples of the natural and organism-derived surfactant include an amphiphilic lipid. The amphiphilic lipid may be a phosphorus-free lipid or a phospholipid, alternatively, may be a cationic lipid, a neutral lipid, an anionic lipid, or a combination thereof.

Examples of the phosphorus-free lipid include N-(2,3-dioleoyloxy-1-propyl)trimethylammonium methyl sulphate (DOTAP), N-[1-(2,3-oleyloxy)propyl]-N,N,N-trimethylammonium chloride (DOTMA), and dioctadecylamide-glycylspermine (DOGS). Examples of the phospholipid include phosphoethanolamines such as 1,2-dioleoyl-sn-glycero-3-phosphoethanolamine (DOPE), 1,2-distearoyl-sn-glycero-3-phosphoethanolamine (DSPE), 1,2-dipalmitoyl-sn-glycero-3-phosphoethanolamine (DPPE), diphytanoyl-sn-glycero-3-phosphoethanolamine (DPhPE); and phosphocholines such as 1,2-dipalmitoyl-sn-glycero-3-phosphocholine (DPPC), 1,2-dimyristoyl-sn-glycero-3-phosphocholine (DMPC), 1,2-distearoyl-sn-glycero-3-phosphocholine (DSPC), and diphytanoyl-sn-glycero-3-phosphocholine (DPhPC).

The amphiphilic molecules may be composed of a surfactant into which a labeling material (e.g., coumarin, a fluorescent molecule such as FITC (fluorescein isothiocyanate), a maleimide group, and a PEG group) has been introduced.

The present nanoparticle preferably includes cholesterol, and the cholesterol is more preferably contained in the membrane of the present nanoparticle. When the present nanoparticle is constituted so as to include cholesterol in its membrane, the fluidity of the membrane of the present nanoparticle is improved compared to a membrane not including cholesterol.

[Other component]

The composition for cryopreservation according to the present embodiment may further contain a component other than the present nanoparticle (hereinafter, also simply referred to as "other component").

Examples of the other component include an encapsulation component encapsulated in the present nanoparticle; and a dispersion medium and amphiphilic molecules or aggregate thereof that have not form a monolayer or bilayer during the process of producing the present nanoparticle.

Encapsulation component

The encapsulation component is a component that is incorporated in the membrane of the present nanoparticle or the surfactant constituting the present nanoparticle or a component that is encapsulated in the inner aqueous phase.

Examples of the encapsulation component include a labeling material (e.g., a fluorescent material such as coumarin), a lipophilic drug, a water-soluble drug, a nucleic acid, a protein, an antibody, a crystalline drug, an organism-derived component, a water-soluble vitamin, a water-soluble vitamin derivative, a lipophilic vitamin, and a lipophilic vitamin derivative.

Specifically, examples of the encapsulation component include active vitamin C and active vitamin A that have a protective effect on cells; and ferulic acid and hyaluronic acid that are water retention components.

The present nanoparticle preferably encapsulates at least one selected from the group consisting of a water-soluble vitamin, a water-soluble vitamin derivative, and ferulic acid and more preferably encapsulates at least one of ferulic acid and active vitamin C.

In particular, since ferulic acid and active vitamin C have excellent antioxidant ability and moisture retention, cell damage due to cell cryopreservation can be more suppressed by the present nanoparticle encapsulating at least one of them.

Dispersion medium

The present nanoparticle is preferably present as a dispersion in a dispersion medium.

Examples of the dispersion medium include water, a buffer solution, a water-soluble solvent, and a liquid culture medium. The dispersion medium may be a single medium or a combination of two or more media.

Examples of the water include distilled water, deionized water, ultrafiltrated water, and pure water.

The content of water is preferably 70 mass% or more based on the total amount of the dispersion medium, and more preferably 80 mass% or more and 90 mass% or less and further preferably 90 mass% or more and 100 mass% or less. The present nanoparticle is more stably present by controlling the content of water within the above-mentioned numerical value range.

Examples of the buffer solution include a phosphate buffer solution (PBS), a trishydroxymethylaminomethane

buffer solution (TRIS), a hydroxyethylpiperazine ethanesulfonic acid buffer solution (HEPES), a borate buffer solution, and an acetate buffer solution.

The concentration of the buffer solution is preferably within a range near to 285 ± 5 m0sm/L, which is human plasma osmotic pressure, depending on the osmotic pressure value.

The pH of the buffer solution is not particularly limited, and is, for example, preferably pH 5 or more and pH 10 or less and more preferably pH 6 or more and pH 9 or less, from the viewpoint of cell viability or the like.

The pH is a value measured at 25°C using a pH meter (the same hereinafter).

The water-soluble solvent is a solvent that is mixable with water.

Examples of the water-soluble solvent include alcohols such as methanol and ethanol from the viewpoint of the solubility of a lipid and so on.

As the water-soluble solvent, for example, the water-soluble solvent used for dissolving the amphiphilic molecules such as a lipid prepared in the manufacturing process of the present nanoparticle may be directly used.

Examples of the liquid culture medium include known culture media that are used in culture of stem cells. More specifically, examples of the liquid culture medium include DMEM (Dulbecco's Modified Eagle's Medium), DMEM: F-12 (Dulbecco's Modified Eagle Medium: Nutrient Mixture F-12), EMEM (Eagle's minimal essential medium), and these liquid culture media supplied with cell growth factors such as platelet-derived growth factor (PDGF), epidermal growth factor (EGF), fibroblast growth factor (FGF), hepatocyte growth factor (HGF), vascular endothelial growth factor (VEGF), insulin-like growth factor (IGF), and nerve growth factor (NGF).

The liquid culture medium may be a commercially available product. Examples of the commercially available product include ADSC-BulletKit (registered trademark) PT-4505 manufactured by LONZA K.K.

The pH (at 25°C) of the liquid culture medium may be, for example, pH 7.0 to 8.0 or pH 7.3 to 7.4.

The pH (at 25°C) of the composition for cell cryopreservation is not particularly limited, and is, for example, preferably pH 4 or more and pH 12 or less and more preferably pH 5 or more and pH 11 or less from the viewpoint of cell viability or the like.

Manufacturing method of composition for cryopreservation

The method for manufacturing the present composition for cryopreservation is not particularly limited, and a known method can be applied.

The method for manufacturing the composition for cryopreservation may be, for example, a manufacturing method including a first step of mixing a solution prepared by dissolving components (materials such as a surfactant (including an encapsulation component when the encapsulation component is encapsulated in the present nanoparticle)) constituting the membrane and so on of the present nanoparticle in a water-soluble solvent and water or a buffer solution to obtain a dispersion including the present nanoparticle and a second step of subjecting the dispersion to filter treatment.

In particular, the particle diameter of the present nanoparticle is adjusted by performing the second step.

(First step)

In the first step, a dispersion including at least one component of the present nanoparticle is obtained. This dispersion can be prepared by mixing a solution of a component constituting the membrane of the present nanoparticle dissolved in a water-soluble solvent (e.g., alcohol) and water or a buffer solution and controlling the conditions (e.g., pH and temperature). Since the amphiphilic molecules constituting the present nanoparticle is as described above, the explanation thereof is omitted here.

For example, a dispersion including a liposome can be prepared by mixing ethanol and a lipid to dissolve the lipid, and mixing and stirring it with water while adjusting the pH.

In the first step, excessive amphiphilic molecules such as cholesterol and phospholipid not contributing to the formation of the present nanoparticle and also, when a component to be encapsulated is present, the unencapsulated component may be removed by a dialysis membrane or the like.

In the second step, the dispersion obtained in the first step is subjected to extrusion treatment by a filter.

Size adjustment of (sizing) the present nanoparticle can be performed by extrusion treatment. The filter may be appropriately selected from commercially available products by choosing a desired aperture size depending on the purpose and so on.

<Cryopreservation method>

The present cryopreservation method includes a step of mixing a cell or biological tissue and the present composition for cryopreservation to introduce the present nanoparticle into a cell (hereinafter, referred to as "introduction step") and a step of freezing the cell or biological tissue into which the present nanoparticle has been introduced

(hereinafter, also referred to as "freezing step"). When biological tissue is frozen, the above "to introduce into a cell" means "to introduce into a cell constituting biological tissue".

According to the present cryopreservation method, cell damage due to cryopreservation is reduced.

The cells as the freezing target of the present cryopreservation method are not particularly limited, and known cells that are cryopreserved can be adopted. Examples of the cells include somatic stem cells such as mesenchymal stem cells, i.e., a human adipose tissue-derived mesenchymal stem cell (hAD-MSC) and a human bone marrow-derived mesenchymal stem cell (hMSC-BM), a neural stem cell, a skin stem cell, a hematopoietic stem cell, a dental pulp stem cell, a liver stem cell, a muscle stem cell, and an adipose stem cell; pluripotent stem cells such as an induced pluripotent stem cell (iPS cell) and an embryonic stem cell (ES cell); blood cells such as a lymphocyte and a neutrophil; organ-specific cells such as an epithelial cell, an endothelial cell, a muscle cell, a fibroblast (such as a skin cell), a smooth muscle cell, a melanocyte, a hair cell, a liver cell, a gastric mucosal cell, an intestinal cell, a spleen cell, and a pancreatic cell (such as an exocrine pancreatic cell); differentiated cells such as a brain cell, a lung cell, a kidney cell, and an adipose cell; and reproductive cells such as an embryo, a fertilized egg, and a sperm. Examples of the freezing target of the present cryopreservation method also include an organoid, a spheroid, a cell sheet, and biological tissue composed of the above cells.

[Introduction step]

In the introduction step, cells or biological tissues and the present composition for cryopreservation are mixed to introduce the present nanoparticle into the cells (cells constituting the biological tissue when biological tissue is frozen, the same hereinafter).

The culture medium is not particularly limited, and examples thereof include known culture media that are used in culturing stem cells. For example, examples of the culture medium include the above-described liquid culture media.

The method for introducing the present nanoparticle into cells is not particularly limited, and examples thereof include the following methods (I) to (IV):

(I) A method in which the present composition for cryopreservation is added to a culture medium containing cells;
(II) A method in which a culture medium containing cells is centrifugated to remove the supernatant and the present composition for cryopreservation is then added thereto;
(III) A method in which cells peeled off from a culture container are added to the present composition for cryopreservation; and
(IV) A method in which the present composition for cryopreservation is introduced into cells using a voltage by electroporation.

[Freezing step]

In the freezing step, the cells or biological tissues into which the present nanoparticle has been introduced is frozen.

The method for freezing a cell or biological tissue is not particularly limited, and a known freezing method can be adopted.

The temperature for freezing a cell or biological tissue is preferably within a range of from -30°C to -196°C (preferably from -80°C to -196°C). The method for freezing a cell or biological tissue is, for example, from the viewpoint of more reducing cell damage due to cryopreservation, preferably freezing a cell or biological tissue using a deep freezer or liquid nitrogen. The freezing period is not particularly limited, and can be 0.5 days or more and 50 years or less, 1 day or more and 10 years or less, 1 week or more and 5 years or less, or 1 month or more and 1 year or less.

[Other step]

The present cryopreservation method may further include a step other than the introduction step and the freezing step. Examples of the other step include a storage step of storing the frozen cell at an appropriate temperature.

[Frozen cell]

The frozen cell or biological tissue according to the present embodiment is a frozen cell or biological tissue including the present nanoparticle. This frozen cell or biological tissue can be produced by the above-described present cryopreservation method.

## Examples

The present invention will be described in more detail below with reference to examples. The materials, the amounts to be used, rates, treatment procedures, and so on shown in the following examples can be appropriately modified within a range not deviating from the gist of the present invention. Accordingly, the scope of the present invention should not be construed as being limited to the specific examples shown below.

<Example 1>

Production of composition for cryopreservation

A double membrane vesicle having DOTAP as a constituent was produced by an organic solvent injection method as Example 1 according to the following procedure:

(1) In a beaker, 5.3 mL of PBS (pH = 7.4 at 25°C) was placed and stirred at 25°C;
(2) In a centrifuge tube, 1990 μL of ethanol, 38.7 mg of cholesterol, and 11.1 mg of DOTAP, which is a lipid, as amphiphilic molecules were mixed, and the temperature of this mixture solution was raised to 75°C, which is not lower than the phase transition temperature of the lipid, to completely dissolve the cholesterol and the lipid. In evaluation of the cell uptake property described below, fluorescence-labeled coumarin was further added, and adjustments and tests were performed under a light-shielded condition in order to prevent attenuation. Coumarin is hydrophobic and is therefore incorporated mainly in a formed membrane of nanoparticles;
(3) The entire amount of the mixture solution was taken in a syringe and was rapidly mixed in the above-mentioned PBS;
(4) The beaker was shielded from light, and stirring was performed at 25°C for 1 hour;
(5) Dialysis was performed at room temperature using a dialysis membrane (Spectra/Por RC Biotech Dialysis Membrane MWCO: 8-10) in PBS for 4 hours or more. Consequently, a solution of nanoparticles was obtained; and
(6) The obtained nanoparticle solution was set in LF-STB LiposoFast Stabilizer manufactured by Avestin, Inc. and was allowed to pass through filters with aperture sizes of 1000 nm, 800 nm, 400 nm, 200 nm, 100 nm, and 50 nm sequentially in this order while applying pressure. Thus, sizing was performed. The obtained liquid composition containing nanoparticles was defined as a composition for cryopreservation of Example 1. The pH (at 25°C) of the composition for cell cryopreservation was 7.2.

<Examples 2 to 5>

Compositions for cryopreservation of Examples 2 to 5 were obtained as in Example 1 except that the type of the amphiphilic molecules was changed from DOTAP to DOPE, Gemini-type, DPPC, or DSPE. "Gemini-type" means "Gemini-type surfactant". The molecular structure of the Gemini-type surfactant used in Example 3 and Examples 8 and 13 described later is shown below. The Gemini-type surfactant is a dimer surfactant having two surfactant units, wherein the hydrophobic group consists of a hydrocarbon chain having 12 carbon atoms, the spacer group has 3 carbon atoms, and the elements forming a pair of ions are bromine and nitrogen.

[Chem 1]

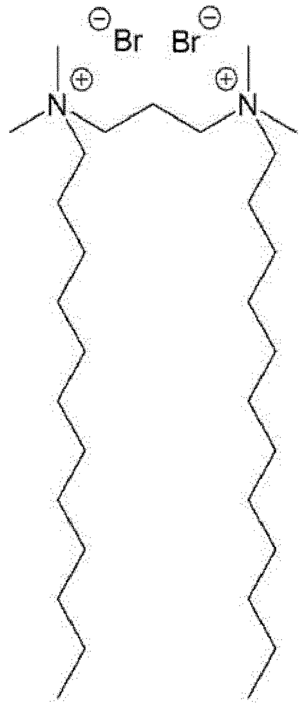

<Examples 6 to 15>

Compositions for cryopreservation of Examples 6 to 15 were obtained by the same method as in Examples 1 to 5 except that the operation (1) of producing the composition for cryopreservation of Example 1 was modified to the operation (1') shown below. Examples 6 to 10 relate to compositions for cryopreservation containing nanoparticles that include DOTAP, DOPE, Gemini-type, DPPC, and DSPE, respectively in this order, as the constituents and contain ferulic acid. Examples 11 to 15 relate to compositions for cryopreservation containing DOTAP, DOPE, Gemini-type, DPPC, and DSPE, respectively in this order, as the constituents and contain active vitamin C.
(1') In a beaker, 5.3 mL of PBS (pH = 7.4 at 25°C) was placed, and 10 μM of ferulic acid or active vitamin C was dissolved therein as an encapsulation component, followed by stirring at 25°C.
The active vitamin C used Examples 11 to 15 is a compound having the following molecular structure.

[Chem 2]

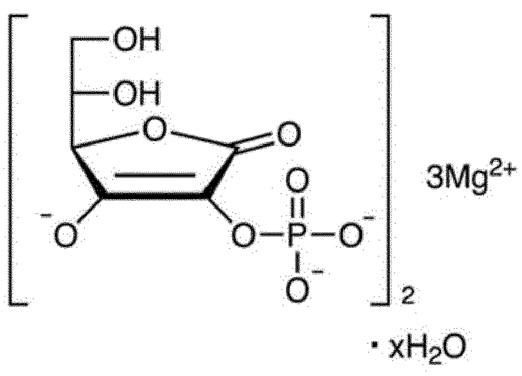

<Comparative Example 1>

In Comparative Example 1, 1 mL of a mixture solution of 10 vol% of dimethyl sulfoxide (DMSO), 40 vol% of fetal

bovine serum (FBS), and 50 vol% of stem cell culture medium was used as the composition for cryopreservation.

<Comparative Example 2>

In Comparative Example 2, CELLBANKER (registered trademark) 1 (1 mL) manufactured by ZNQ not including a nanoparticle was used as the composition for cryopreservation.

[Test Example 1]

<Volume average particle diameter and zeta-potential>

The volume average particle diameter and zeta-potential of the composition for cryopreservation of each Example were measured. Specifically, 1 mL of a solution of each of the compositions for cryopreservation of Examples 1 to 5 each diluted 100 times with phosphate buffered saline (PBS, pH 7.4) was collected and was filled in a 1-mL cell for measurement. This solution was subjected to measurement with a particle size and zeta-potential analyzer (ZETASIZER PRO, manufactured by Malvern) at room temperature (25°C). The results are shown in Table 1. Figure 1 shows the distribution of particle diameters of a nanoparticle included in the composition for cryopreservation of Example 1.

[Table 1]

| | **Cationic nanoparticle** | | | **Neutral nanoparticle** | |
|---|---|---|---|---|---|
| | **Example 1** | **Example 2** | **Example 3** | **Example 4** | **Example 5** |
| **Particle composition (main component)** | **DOTAP** | **DOPE** | **Gemini-type** | **DPPC** | **DSPE** |
| **Particle diameter size range (nm)** | 20.8-307 | 31.4-296 | 19.7-271 | 39.7-304 | 52.2-286 |
| **Average particle diameter (nm)** | 111 | 109 | 105 | 114 | 108 |
| **Z-potential (mV)** | 36.8 | 30.4 | 42.5 | -0.0076 | -0.0541 |

[Test Example 2]

<Evaluation of cell uptake property>

The cell uptake property of the nanoparticle included in each of the compositions for cryopreservation of Examples 1 to 5 was evaluated by the following procedure:

(1) A human adipose tissue-derived mesenchymal stem cell (hAD-MSC) after freezing and thawing was seeded in a collagen-coated 8-well chamber slide at 2000 cells/well;
(2) The compositions for cryopreservation of Examples 1 to 5 labeled with fluorescence dye coumarin were added to the wells and were incubated at 37°C for 30 minutes;
(3) Cell nuclei were dyed using a DAPI solution (4',6-diamidino-2-phenylindole dihydrochloride solution); and
(4) The fluorescence intensities of cytoplasm and cell nuclei observed with a fluorescence microscope (manufactured by KEYENCE Corporation, BZ-9000) at a magnification of 20 times, an exposure time of 1/2 seconds, and black balance 42 were each measured. The values of the ratio of the fluorescence intensity IN of coumarin encapsulated in the nanoparticles present in cell nuclei to the fluorescence intensity OUT of coumarin encapsulated in nanoparticles present in a region within the cytoplasm and outside the cell nuclei, (IN/OUT), are shown in the paragraph of the evaluation of cell uptake property [Fluorescence intensity ratio (cell nucleus/cytoplasm)] in Table 2.

[Table 2]

| | **Cationic nanoparticle** | | | **Neutral nanoparticle** | |
|---|---|---|---|---|---|
| | **Example 1** | **Example 2** | **Example 3** | **Example 4** | **Example 5** |
| **Particle composition (main component)** | **DOTAP** | **DOPE** | **Gemini-type** | **DPPC** | **DSPE** |

(continued)

| | Cationic nanoparticle | | | Neutral nanoparticle | |
|---|---|---|---|---|---|
| | Example 1 | Example 2 | Example 3 | Example 4 | Example 5 |
| **Fluorescence intensity ratio (nucleus/cytoplasm)** | 0.348 | 0.311 | 0.425 | 0.105 | 0.114 |

As shown in Table 2, since the values of the fluorescence intensity ratio (cell nucleus/cytoplasm) were positive numbers, it was demonstrated that some of the nanoparticles included in each of the compositions for cryopreservation of Examples 1 to 5 was introduced into the cell nucleus without remaining in the cytoplasm.

[Test Example 3]

<Transmission electron microscope>

The nanoparticles introduced into cells by the same method as in Test Example 2 were observed with a transmission electron microscope. Adipose tissue-derived mesenchymal stem cells (hAD-MSC) of a healthy subject were seeded in a 3.5 $cm^2$ petri dish at a density of about 5,000 cells/$cm^2$ using 5 mL of a growth factor-containing special culture medium. The composition for cryopreservation of Example 3 (Gemini-type) was labeled using gold nanoparticle (Cytodiagnostics Inc.). The labeled composition for cryopreservation was exposed to ADSC for 30 minutes. In order to prepare a sample for transmission electron microscopic observation, as prefixation, ADSC was fixed using 2% glutaraldehyde-containing phosphate buffer solution at 4°C overnight. The fixed sample was washed with a phosphate buffer solution and was then fixed using a 2% osmium aqueous solution at 4°C for 2 hours as postfixation. The concentration of ethanol was increased from 30% to 100% in a stepwise manner, and treatment was performed for 15 minutes at each concentration for dehydration. The sample was embedded in an epoxy resin over 48 hours at 60°C, and an ultra-thin section of 80 to 90 nm was produced therefrom using an ultramicrotome and was loaded on a Cu 200 mesh. Observation was performed using a transmission electron microscope H-7600 at 100 kV. The results are shown in Figure 2.

As shown in Figure 2, nanoparticles included in the composition for cryopreservation of Example 3 were incorporated in the cytoplasm. Furthermore, some of nanoparticles having a small particle diameter was also incorporated in the nuclei.

[Test Example 4]

<Cryopreservation and thawing treatment of cell using composition for cryopreservation>

Cryopreservation of cell

(1) 70% to 90% confluent human adipose tissue-derived mesenchymal stem cells (hAD-MSC) were trypsinized, peeled off from the culture container, and centrifugated to obtain a cell palette.
(2) Ten microliter of the composition for cryopreservation of each Example was added to and introduced into about $5 \times 10^6$ cells described above, and 990 $\mu L$ of the cell culture medium was added thereto to obtain a cell suspension. In Comparative Examples, 1 mL of the composition for cryopreservation of each Example was added to the cells to obtain a cell suspension.
(3) The above cell suspensions were dispensed in cryo tubes and were cryopreserved in a deep freezer of -80°C.

Thawing of cryopreserved cell

(1) The cryopreserved (within 1 month) cells contained in the cryo tubes were quickly thawed by shaking the tubes in a warm bath of 37°C.
(2) The thawed cell suspension was mixed with 10 mL of a stem cell special culture medium to obtain a mixture solution.
(3) The mixture solution was centrifugated (1,200 rpm, 5 minutes, 4°C), the supernatant was removed using an

aspirator, and the cells were then suspended in an appropriate amount of the above culture medium.

<Evaluation of cell survival rate>

The cell survival rate before and after the cell cryopreservation was evaluated using a cell proliferation assay kit (MTT Cell Viability Assay Kit, AR1156, BTI, manufactured by Cosmo Bio Co, Ltd.) by the following procedure.

(1) Cells thawed after cryopreservation using the compositions for cryopreservation of Examples 3 and 6 to 15 were seeded in a 96-well plate at 2,000 cells/well.
(2) To each well, 10 μL of the MTT standard reagent in the kit was added, followed by incubation at 37°C for 4 hours.
(3) To each well, 100 μL of a formazan solution was added, followed by incubation at 37°C for 18 hours.
(4) The absorbance A of the dye at an absorbance wavelength of 570 nm was measured using a microplate absorbance reader.
(5) The above operations (1) to (3) were performed using hMSC-AT before cell freezing, and the absorbance B of the dye at an absorbance wavelength of 570 nm was measured using a microplate absorbance reader.
(6) The rate of the absorbance A after thawing obtained in the above (4) to the absorbance B before cryopreservation (= A/B × 100%) was defined as the cell survival rate. The results are shown in Tables 3 and 4.

[Table 3]

| | Neutral nanoparticle | | Cationic nanoparticle | | | | Comparative Example | |
|---|---|---|---|---|---|---|---|---|
| | Example 9 | Example 10 | Example 6 | Example 7 | Example 8 | Example 3 | Comparative Example 1 | Comparative Example 2 |
| Particle composition (main component) | DPPC | DSPE | DOTAP | DOPE | Gemini-type | Gemini-type | 10% DMSO | CELLBANKER |
| Encapsulated material | Ferulic acid | | | | | None | | |
| Survival rate after freezing (%) | 73.8 | 75.4 | 88.5 | 81.7 | 94.5 | 86.8 | 69.8 | 71.5 |

[Table 4]

| | Neutral nanoparticle | | Cationic nanoparticle | | | | Comparative Example | |
|---|---|---|---|---|---|---|---|---|
| | Example 14 | Example 15 | Example 11 | Example 12 | Example 13 | Example 3 | Comparative Example 1 | Comparative Example 2 |
| **Particle composition (main component)** | **DPPC** | **DSPE** | **DOTAP** | **DOPE** | **Gemini-type** | **Gemini-type** | **10% DMSO** | **CELLBANKER** |
| **Encapsulated material** | **Active vitamin C** | | | | | **None** | | |
| **Survival rate after freezing (%)** | 77.1 | 78.3 | 84.1 | 87.9 | 95.8 | 89.5 | 68.7 | 72.4 |

As shown in Tables 3 and 4, it was demonstrated that when freezing is performed using the composition for cryopreservation of Example 3 and the compositions for cryopreservation encapsulating ferulic acid or active vitamin C of Examples 6 to 15, the cell survival rate is high, that is, the cell damage due to cryopreservation is reduced, compared to when freezing is performed using the composition for cryopreservation of Comparative Example.

[Test Example 5]

<Evaluation of stem cell rate>

Cells were cryopreserved using the compositions for cryopreservation of Examples 3 and 6 to 15, and the stem cell rates thereof were evaluated using Human Mesenchymal Stem Cell Multi-Color Flow Kit (FMC 020, manufactured by R&D systems, Inc.) by the following procedure. The kit uses CD90 as a positive marker and CD45, CD34, CD11b, CD79A, and HLA-DR as negative markers.

(1) $5 \times 10^6$ cells thawed after cryopreservation using the compositions for cryopreservation of Examples 3 and 6 to 15 were mixed with 100 μL of dye-buffer solution for flow cytometry associated with the kit, and the mixture was transferred to the tube for flow cytometry to provide a cell suspension.
(2) To the cell suspension, 10 μL of each of solutions of two fluorescence dye-labeled antibodies (one antibody solution containing a positive marker and one antibody solution containing a negative marker) associated with the kit was added, followed by incubation at room temperature (25°C) in dark for 45 minutes.
(3) The cell suspension after the incubation was washed with 2 mL of the dye buffer solution for flow cytometry.
(4) Flow cytometry was performed using a flow cytometer to quantitatively measure the cells exhibiting characteristics of CD90(+), CD45(-), CD34(-), CD11b(-), CD79A(-), or HLA-DR(-) after the cell cryopreservation. The rates of the cells when cryopreservation was performed using the compositions for cryopreservation of Examples 3 and 6 to 15 are shown as "stem cell rate" in Tables 5 and 6.

As shown in Tables 5 and 6, it was demonstrated that when cryopreservation was performed using the compositions for cryopreservation including nanoparticles of Examples 3 and 6 to 15, the rate of stem cells was high, compared to when the composition for cryopreservation of Comparative Example was used, that is, the compositions are useful for maintaining the stem cell function through the cryopreservation.

[Table 5]

| | Neutral nanoparticle | | Cationic nanoparticle | | | | Comparative Example | |
|---|---|---|---|---|---|---|---|---|
| | Example 9 | Example 10 | Example 6 | Example 7 | Example 8 | Example 3 | Comparative Example 1 | Comparative Example 2 |
| Particle composition (main component) | DPPC | DSPE | DOTAP | DOPE | Gemini-type | Gemini-type | 10% DMSO | CELLBANKER |
| Encapsulated material | Ferulic acid | | | | | None | | |
| Stem cell rate after freezing (%) | 73.4 | 69.8 | 84.5 | 81.4 | 90.3 | 85.3 | 59.1 | 69.8 |

[Table 6]

| | Neutral nanoparticle | | Cationic nanoparticle | | | | Comparative Example | |
|---|---|---|---|---|---|---|---|---|
| | Example 14 | Example 15 | Example 11 | Example 12 | Example 13 | Example 3 | Comparative Example 1 | Comparative Example 2 |
| Particle composition (main component) | DPPC | DSPE | DOTAP | DOPE | Gemini-type | Gemini-type | 10% DMSO | CELLBANKER |
| Encapsulated material | Active vitamin C | | | | | None | | |
| Stem cell rate after freezing (%) | 72.5 | 66.8 | 82.7 | 82.6 | 90.9 | 83.4 | 58.7 | 67.1 |

[Test Example 6]

<Comparison of Gemini-type surfactant>

In the above test, the compositions for cryopreservation of Examples 3, 8, and 13 were used. That is, a Gemini-type surfactant including hydrophobic groups each consisting of a hydrocarbon chain having 12 carbon atoms, a spacer group having 3 carbon atoms, and bromine and nitrogen as the elements forming a pair of ions was used as the Gemini-type surfactant. Accordingly, the compositions for cryopreservation of Examples 16 to 19 were produced by the same method as in Example 1 except that the Gemini-type surfactant was changed in the number of carbon atoms of the two alkyl chains, the number of carbon atoms of the spacer, and the elements that form a pair of ions, and the survival rate after freezing was investigated by the same method as in Test Example 4 together with the composition for cryopreservation of Example 3. The results are shown in Table 7. In Table 7, 12-3-12_Br means that the number of carbon atoms of each of two hydrocarbon chains is 12, the number of carbon atoms of the spacer is 3, and the elements forming a pair of ions are bromine and nitrogen; and 16-3-16_Cl means that the number of carbon atoms of each of two hydrocarbon chains is 16, the number of carbon atoms of the spacer is 3, and the elements forming a pair of ions are chlorine and nitrogen.

[Table 7]

| | **Gemini-type Cationic nanoparticle** | | | | |
|---|---|---|---|---|---|
| | **Example 3** | **Example 16** | **Example 17** | **Example 18** | **Example 19** |
| **Particle composition (main component)** | **12-2-12_Br** | **12-3-12_Br** | **12-3-12_Cl** | **16-3-16_Br** | **16-3-16_Cl** |
| **Survival rate after freezing (%)** | 76.5 | 85.6 | 78.2 | 75.8 | 82.1 |

As shown in Table 7, even when cells were frozen using a composition for cryopreservation including a nanoparticle composed of a Gemini-type surfactant of which the hydrocarbon chains each having 16 carbon atoms not only 12 carbon atoms, a high survival rate was maintained. In addition, even when chlorine was used instead of bromine as the element that forms a pair of ions in the Gemini-type surfactant, a high survival rate was maintained. Furthermore, a high survival rate was maintained in both Gemini-type surfactants in which the spacer had 2 or 3 carbon atoms.

[Test Example 7]

<Long cryopreservation>

The composition for cryopreservation of Example 3 was introduced into cells by the same method as in Test Example 4, followed by freezing. The survival rates of the cells after thawing when cryopreserved for 3, 6, or 12 months were investigated by the same method as in Test Example 4. The results are shown in Table 8.

[Table 8]

| | **Gemini-type Cationic nanoparticle** | | | |
|---|---|---|---|---|
| | **Example 3 (12-3-12_Br)** | | | |
| **Storage period (month)** | **0** | **3** | **6** | **12** |
| **Survival rate after freezing (%)** | 87.6 | 88.1 | 87.9 | 87.8 |

As shown in Table 8, even when the composition for cryopreservation of Example 3 was introduced into the cells and the cells were cryopreserved for 12 months, a survival rate of 87.8% was maintained even after freezing-thawing.

[Test Example 8]

<Extracellular discharge of composition>

The composition for cryopreservation of Example 3 was introduced into cells by the same method as in Test Example 2 and frozen. The fluorescence intensities in the cytoplasm and nucleus were quantitatively measured 0, 1, 7, 14 days after the thawing to investigate extracellular discharge of the introduced composition for cryopreservation. The

results are shown in Table 9.

[Table 9]

| | Gemini-type Cationic nanoparticle | | | |
|---|---|---|---|---|
| | Example 3 (12-3-12_Br) | | | |
| **Time after thawing (day)** | **0** | **1** | **7** | **14** |
| **Fluorescence intensity (the entire cell)** | 0.753 | 0.565 | 0.066 | 0.001 |

As shown in Table 9, the fluorescence intensity was one-tenth or less 7 days after thawing and was decreased to an almost undetectable level 14 days after thawing. Accordingly, it was confirmed that in freezing with the composition for cryopreservation of Example 3, the composition for cryopreservation underwent extracellular discharge after thawing.

[Test Example 9]

<Doubling time of cell after thawing>

The composition for cryopreservation of Example 3 was introduced into cells by the same method as in Test Example 4 and frozen. The time took for the total number of cells to double as a result of cell proliferation of the cells 0 or 7 days after thawing (doubling time of cells: PDT (Population Doubling Time)) was measured. The results are shown in Table 10.

The doubling time was calculated by the following equation. The computation method for PDT (Population Doubling Time):

1) Cells were counted at two points in the logarithmic proliferation stage.
2) The measurement values of the cell density at the first point and the second point were defined as N0 and N, respectively.

The times from the start of culture until the first point and the second point were defined as t0 and Ht, respectively.

$$PDT = (t - t0)\log 2/(\log N - \log N0)$$

[Table 10]

| | Gemini-type Cationic nanoparticle | |
|---|---|---|
| | Example 3(12-3-12_Br) | |
| **Start time of doubling time measurement after thawing (Nth day)** | **0** | **7** |
| **Doubling time (hr)** | 63 | 62 |

As shown in Table 10, the doubling time of cells was maintained even 7 days after thawing. Accordingly, it was confirmed that even when cells were frozen using the composition for cryopreservation of Example 3, the proliferative activity of the cells was not influenced by cryopreservation.

[Test Example 10]

<Survival rate of embryo, sperm, blood cell, and 3D organoid after freezing>

The composition for cryopreservation of Example 3 was introduced into cells by the same method as in Test Example 4. As cell species, in addition to human adipose tissue-derived mesenchymal stem cells, embryos, sperms, blood cells, and 3D organoids were used. The survival rates after freezing were investigated by the same method as in Test Example 4. The results are shown in Table 11.

The used cells were as follows:

Embryo (species: mouse, strain: C57BL/6J Jcl, stage: 2 cells, number of embryos: 30, purchased from: National Institutes of Biomedical Innovation, Health and Nutrition);
Sperm (species: mouse, strain: C57BL/6NCr Slc, number of sperms: 1 straw, purchased from: National Institutes of Biomedical Innovation, Health and Nutrition);
Blood cell (species: human, source: peripheral blood from an acute monocytic leukemia patient, type: monocyte, purchased from: ATCC); and
3D organoid (species: human, source: healthy adipose tissue, type: mesenchymal stem cell, organoid culture using Corning (registered trademark) Matrigel basement membrane matrix for organoid culture, purchased from: LONZA K.K.).

[Table 11]

| | **Gemini-type Cationic nanoparticle** | | | | | **10% DMSO** | **CELLBANKER** |
|---|---|---|---|---|---|---|---|
| | **Example 3 (12-3-12_Br)** | | | | | **Comparative Example 1** | **Comparative Example 2** |
| **Cell species** | **Embryo** | **Sperm** | **Blood cell** | **3D organoid** | **Human adipose tissue-derived mesenchymal stem cell** | | |
| **Survival rate after freezing (%)** | 86.5 | 81.7 | 89.5 | 86.8 | 88.2 | 58.4 | 69.1 |

As shown in Table 11, it was confirmed that high survival rates could be maintained by using the composition for cryopreservation of Example 3 even when cells such as embryos, sperms, and blood cells and 3D organoids were frozen as in mesenchymal stem cells.

[Test Example 11]

<Evaluation of transplantation efficiency in immunodeficient mouse>

The composition for cryopreservation of Example 8 was introduced into mesenchymal stem cells by the same method as in Test Example 4, and the cells were frozen and thawed and were used for evaluation of transplantation efficiency in immunodeficient mice.

Matrigel for spheroid culture and a growth factor included in a special culture medium were added to adipose tissue-derived mesenchymal stem cells (ADSC) of a healthy subject, and culture of the cells in a spheroid form was started one week before transplantation. The stem cell spheroid was mixed with Matrigel for transplantation in a ratio of 1:1. A syringe was filled with a mixture solution for transplantation containing $1 \times 10^7$ cells of ADSC on ice, and the cells were transplanted subcutaneously in the back of immunodeficient mice. The length, width, and height of the transplantation site 2 weeks after the transplantation were measured with a caliper, and the volume ($mm^3$) was calculated. The weight (mg) of the implant extracted 3 weeks after the transplantation of ADSC was weighed using a precision electronic balance. As a control, ADSC frozen without using the composition of Example 8 was used. The results are shown in Table 12.

[Table 12]

| | **Control** | **Gemini-type Cationic nanoparticle** |
|---|---|---|
| | | **Example 8** |
| **Volume of implant ($mm^3$)** | 1250 | 2020 |
| **Weight of implant (mg)** | 1060 | 2420 |

As shown in Table 12, cells treated with the composition for cryopreservation of Example 8 maintained the bioactivity even when transplanted in mice, and an increase in the implant was confirmed.

[Test Example 12]

<In vitro evaluation of carcinogenicity>

As an indicator of safety, in vitro evaluation of carcinogenicity, soft-agar colony formation assay, was performed using the composition for cryopreservation of Example 3.
A 1.8% agar solution was sterilized and dissolved in an autoclave and was kept warm in a water bath of 45°C.
A bottom mix (2 × DMEM: 37.5 mL, serum: 9 mL, 2.8% $NaHCO_3$: 5.25 mL, 10 × penicillin/streptomycin: 0.9 mL) was produced and was kept warm in a water bath of 45°C.
The sufficiently warmed bottom mix and a 1.8% agar solution were mixed in a sterilized reagent bottle or tube in a ratio of 1.4 vol:1 vol to make a total volume of 2.5 mL × required number of sheets + $\alpha$, and were kept warm in a water bath of 45°C (final: 0.75% agar).
In a 35-mm dish or a 6-well plate, 2.5 mL of the above bottom agar was poured each using a disposable pipette, left to stand at 4°C for 8 minutes for hardening, and then kept warm in an incubator.
The sufficiently warmed top mix (upper layer: 2 × DMEM: 37.5 mL, serum: 9 mL, 2.8% $NaHCO_3$: 5.25 mL, 10 × penicillin/streptomycin: 0.9 mL, sterilized water: 19.5 mL) and a 1.8% agar solution were mixed in a sterilized reagent bottle or tube in a ratio of 4 vol:1 vol to make a total volume of 1.5 mL × required number of sheets + $\alpha$, and were kept warm in a water bath of 45°C (final: 0.36% agar).
The human adipose tissue-derived mesenchymal stem cells were peeled off by a usual procedure, then allowed to pass through a cell strainer to obtain single cells, and adjusted to $2 \times 10^5$ cells/mL.
One hundred microliter of the cell suspension was transferred to a 15-mL tube, and the top agar was added thereto in 3 mL/tube using a disposable pipette. Pipetting was performed such that no foaming occurs, and 1.5 mL thereof was sowed each on the bottom agar.
The resulting agar was left to stand at 4°C for 8 minutes for hardening, and was then kept warm in an incubator.
After 2 weeks, the colonies were stained and counted. The results are shown in Table 13.

[Table 13]

| | **Gemini-type Cationic nanoparticle** | | |
|---|---|---|---|
| | **Example 3 (12-3-12_Br)** | | |
| **Storage period (month)** | **0** | **3** | **6** |
| **Number of colonies** | 0 | 0 | 0 |

As shown in Table 13, no colonies were formed during storage periods of 0, 3, and 6 months, and no carcinogenicity by introduction of the composition for cryopreservation of Example 3 into cells was confirmed.

## Claims

1. A composition for cryopreservation of cells or biological tissues, comprising a nanoparticle that comprises amphiphilic molecules as a constituent, wherein the amphiphilic molecules form a monolayer or bilayer.

2. The composition for cryopreservation according to claim 1, wherein the nanoparticle is a cationic nanoparticle.

3. The composition for cryopreservation according to claim 1 or 2, wherein the amphiphilic molecules are a Gemini-type surfactant.

4. The composition for cryopreservation according to claim 1 or 2, wherein the nanoparticle has a zeta-potential of 10 mV or more and 50 mV or less.

5. The composition for cryopreservation according to claim 1 or 2, wherein the nanoparticle has a volume average particle diameter of 30 nm or more and 300 nm or less.

6. The composition for cryopreservation according to claim 1 or 2, wherein the nanoparticle encapsulates ferulic acid or activated vitamin C.

7. A method for cryopreserving cells or biological tissues, comprising the steps of:

   mixing cells or biological tissues with the composition for cryopreservation according to any one of claims 1 to 6 to

introduce a nanoparticle into the cells or biological tissues; and
freezing the cell or biological tissue into which the nanoparticle has been introduced.

**8.** A frozen cell or biological tissue comprising a nanoparticle that comprises amphiphilic molecules as a constituent, wherein the amphiphilic molecules form a monolayer or bilayer.

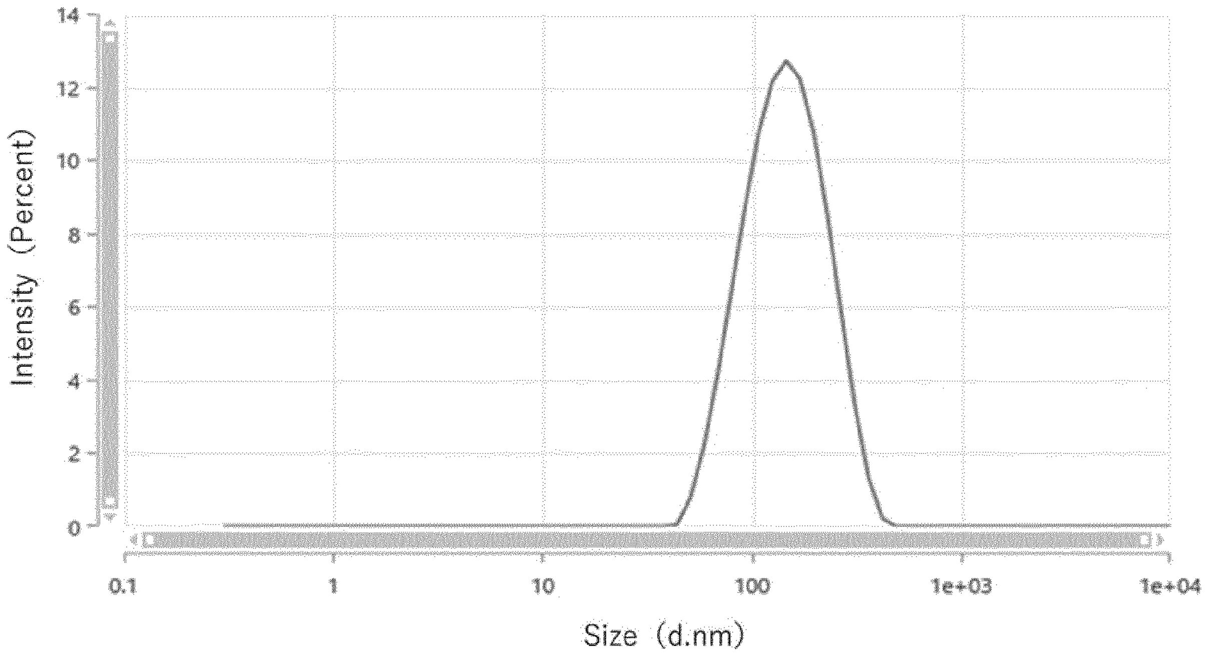
Intensity (Percent)
14
12
10
8
6
4
2
0
0.1
1
10
100
1e+03
1e+04
Size (d.nm)

FIG. 1

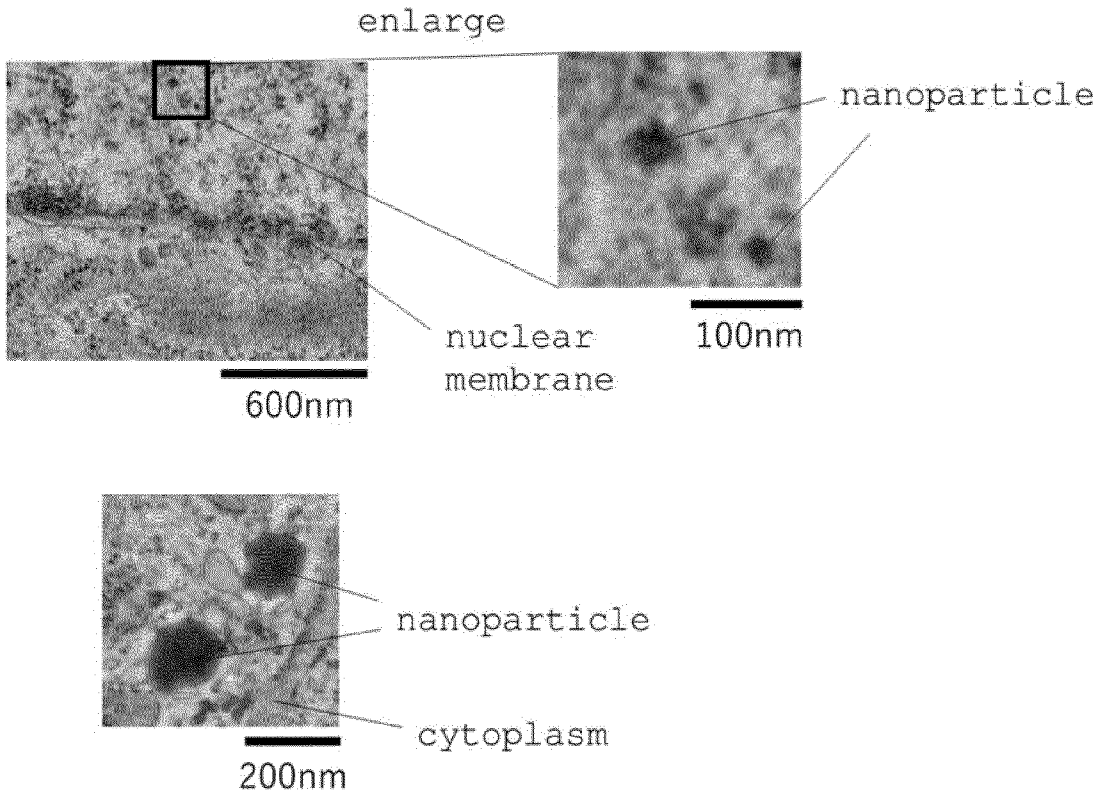
enlarge
nanoparticle
nuclear membrane
600nm
100nm
nanoparticle
cytoplasm
200nm

FIG. 2

## INTERNATIONAL SEARCH REPORT

International application No.
**PCT/JP2023/027582**

**A. CLASSIFICATION OF SUBJECT MATTER**

***C12N 1/04***(2006.01)i; ***C12N 5/00***(2006.01)i
FI: C12N1/04; C12N5/00

According to International Patent Classification (IPC) or to both national classification and IPC

**B. FIELDS SEARCHED**

Minimum documentation searched (classification system followed by classification symbols)

C12N1/04; C12N5/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | NAJAFI, A. et al. Lycopene-loaded nanoliposomes improve the performance of a modified Beltsville extender broiler breeder roosters. Animal Reproduction Science. 2018, vol. 195, pages 168-175<br>abstract, results | 1-8 |
| Y | abstract, results | 6 |
| X | TAOUZINET, L. et al. Alpha Tocopherol loaded in Liposome: Preparation, Optimization, Characterization and Sperm Motility Protection. Drug Delivery Letters. 2020, vol. 10, pages 228-236<br>abstract, page 233, table 4, page 234, fig. 7 | 1-8 |
| Y | abstract, page 233, table 4, page 234, fig. 7 | 6 |
| X | NAJAFI, A. et al. Improvement of post-thawed sperm quality in broiler breeder roosters by ellagic acid-loaded liposomes. Poultry Science. 2019, vol. 98, pages 440-446<br>abstract, results | 1-8 |
| Y | abstract, results | 6 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 October 2023** | **10 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/027582**

**C. DOCUMENTS CONSIDERED TO BE RELEVANT**

| Category* | Citation of document, with indication, where appropriate, of the relevant passages | Relevant to claim No. |
|---|---|---|
| X | JP 2017-500893 A (ESSENTIAL PHARMACEUTICALS, LLC) 12 January 2017 (2017-01-12)<br>claims, paragraphs [0011], [0012], [0083], [0102], [0107], examples | 1-5, 7, 8 |
| Y | claims, paragraphs [0011], [0012], [0083], [0102], [0107], examples | 6 |
| X | US 2006/0188867 A1 (ACKER, Jason P.) 24 August 2006 (2006-08-24)<br>claims, examples | 1-5, 7, 8 |
| Y | claims, examples | 6 |
| X | JP 2002-535349 A (THE BRD. OF TRUSTEES OF THE UNIVERSITY OF ILLINOIS) 22 October 2002 (2002-10-22)<br>claims, paragraph [0057], example 15 | 1, 5, 7, 8 |
| Y | claims, paragraph [0057], example 15 | 6 |
| X | CN 114190367 A (SONGSHAN LAKE MATERIAL LAB.) 18 March 2022 (2022-03-18)<br>claims, paragraph [0012], examples | 1-5, 7, 8 |
| Y | claims, paragraph [0012], examples | 6 |
| X | JP 2015-100287 A (JAPAN ADVANCED INST. OF SCIENCE & TECHNOLOGY HOKURIKU) 04 June 2015 (2015-06-04)<br>claims, paragraphs [0016], [0023], examples | 1, 5, 7, 8 |
| Y | claims, paragraphs [0016], [0023], examples | 6 |
| X | JP 2015-501140 A (UNIV. OF WARWICK) 15 January 2015 (2015-01-15)<br>claims, paragraphs [0017], [0021], examples | 1, 5, 7, 8 |
| Y | claims, paragraphs [0017], [0021], examples | 6 |

Form PCT/ISA/210 (second sheet) (January 2015)

**INTERNATIONAL SEARCH REPORT**
**Information on patent family members**

International application No.
**PCT/JP2023/027582**

| Patent document cited in search report | Publication date (day/month/year) | Patent family member(s) | Publication date (day/month/year) |
|---|---|---|---|
| JP 2017-500893 A | 12 January 2017 | WO 2015/095651 A1<br>claims, paragraphs [0011], [0012], [0086], [0105], [0110], examples<br>US 2015/0175956 A1 | |
| US 2006/0188867 A1 | 24 August 2006 | WO 2006/079205 A1<br>claims, examples | |
| JP 2002-535349 A | 22 October 2002 | WO 00/44348 A2<br>claims, page 28, line 25 to page 29, line 12, example 15 | |
| CN 114190367 A | 18 March 2022 | (Family: none) | |
| JP 2015-100287 A | 04 June 2015 | (Family: none) | |
| JP 2015-501140 A | 15 January 2015 | WO 2013/050773 A1<br>claims, page 4, lines 11-15, page 5, lines 9-16, examples | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5773347 B